(19) Europäisches Patentamt
European Patent Office
Office européen des brevets

(11) Veröffentlichungsnummer: **0 558 569 B1**

# (12) EUROPÄISCHE PATENTSCHRIFT

(45) Veröffentlichungstag der Patentschrift: **25.01.95**

(51) Int. Cl.$^{6}$: **C07C 311/04**, C07C 311/13, C07C 311/17, C07C 311/40, C07C 311/46, A61K 31/18

(21) Anmeldenummer: **91920295.2**

(22) Anmeldetag: **19.11.91**

(86) Internationale Anmeldenummer:
**PCT/EP91/02186**

(87) Internationale Veröffentlichungsnummer:
**WO 92/09571 (11.06.92 92/13)**

(54) **SULFONAMIDE ENTHALTENDE ARZNEIMITTEL, NEUE SULFONAMIDE UND VERFAHREN ZU IHRER HERSTELLUNG.**

(30) Priorität: **22.11.90 DE 4037174**

(43) Veröffentlichungstag der Anmeldung:
**08.09.93 Patentblatt 93/36**

(45) Bekanntmachung des Hinweises auf die Patenterteilung:
**25.01.95 Patentblatt 95/04**

(84) Benannte Vertragsstaaten:
**AT BE CH DE DK ES FR GB GR IT LI LU NL SE**

(56) Entgegenhaltungen:
**EP-A- 0 004 011**
**EP-A- 0 325 245**
**FR-A-23 095 24**
**EP-A- 0 255 728**
**FR-A- 2 193 216**
**US-A- 3 737 316**

**CHEMICAL ABSTRACTS, Band 11, Nr. 25, 18. Juni 1990, Columbus, Ohio, USA; T. IWAKUMA et al, "Phenoxyacetic acids as platelet aggregation inhibitors", Zusammenfassung Nr. 234 980c, Seite 589, Spalte 2; & JP-A-1 279 826**

(73) Patentinhaber: **BOEHRINGER MANNHEIM GMBH**

**D-68298 Mannheim (DE)**

(72) Erfinder: **HECK, Reinhard**
**Ulmenweg 12**
**D-6718 Grünstadt (DE)**
Erfinder: **DRESEL, Hans, Alois**
**Mozartstr. 1**
**D-6905 Schriesheim (DE)**
Erfinder: **HECK, Reinhard**
**Ulmenweg 12**
**D-6718 Grünstadt (DE)**

(74) Vertreter: **Weber, Manfred, Dr. et al**
**c/o Boehringer Mannheim GmbH,**
**Patentabteilung,**
**Sandhoferstrasse 116**
**D-68298 Mannheim (DE)**

**EP 0 558 569 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 106, Nr. 11, 16. März 19897, Columbus, Ohio, USA; I. LINAN CASTELLET, "Process for the preparation of 4-[2-(phenylsulfonylamino)ethyl]phenoxacetic acid", Seite 568, Spalte 2, Zusammenfassung Nr. 84172A; & ES-A-549 348

CHEMICAL ABSTRACTS, Band 112, Nr. 6, 5. Februar 1990, Columbus, Ohio, USA; K. NAKAMURA et al, "Recording materials containing sulfonamide compound as electron-accepting compound", Seite 701, Spalte 1, Zusammenfassung Nr. 45825v; & JP-A-1141786

CHEMICAL ABSTRACTS, Band 113, Nr. 24, 10. Dezember 1990, Columbus, Ohio, USA; T. NAKAMURA et al, "N-(Hydroxyphenyl)sulfonamides as developers for thermal and pressure-sensitive recording materials", Seite 636, Spalte 1, Zusammenfassung Nr. 221 444b; & JP-A-2 145 560

## Beschreibung

Die vorliegende Erfindung betrifft Arzneimittel, die Sulfonamide der allgemeinen Formel I

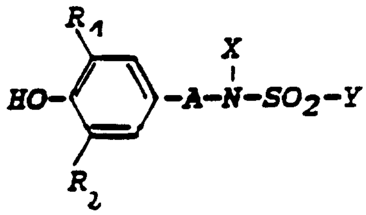

I

in der

$R_1$ und $R_2$, die gleich oder verschieden sein können,

ein Wasserstoffatom oder einen geradkettigen oder verzweigten Alkylrest mit 1 bis 4 C-Atomen,

A eine geradkettige oder verzweigte Alkylenkette mit 1 bis 5 C-Atomen,

X ein Wasserstoffatom, oder einen $C_1$-$C_4$-Alkylrest,

und

Y einen geradkettigen oder verzweigten Alkylrest mit 1 bis 5 C-Atomen, einen Aralkyl- oder Arylrest, wobei der Arylrest ein- bis dreifach in allen möglichen Positionen am Ring durch Halogen, Trifluormethyl, $C_1$-$C_4$-Alkyl, Amino, $C_1$-$C_4$-Acylamino, Di($C_1$-$C_4$)-Alkylamino, oder Nitro substituiert sein kann,

bedeuten,

mit der Maßgabe, daß A auch Valenz bedeuten kann, wenn Y keinen Arylrest darstellt,

sowie deren pharmakologisch unbedenkliche Salze enthalten.

$R_1$ und $R_2$ bedeuten bevorzugt Wasserstoff, Methyl oder die tert-Butylgruppe.

Als "Brücke" A ist bevorzugt $-CH_2-$; $-CH_2-CH_2-$, oder die $-CH_2-CH(CH_3)-$, $-CH_2-C(CH_3)_2-$

X stellt bevorzugt ein H-Atom oder eine Methylgruppe dar.

Eine Aralkylgruppe bedeutet vorzugsweise Benzyl oder Phenethyl.

Eine Arylgruppe stellt vorzugsweise Phenyl dar.

Für Y ist besonders bevorzugt der Isopropyl-, der n-Butyl-, der Benzyl- oder der Phenylrest, wobei dieser seinerseits in allen Positionen ein- bis dreifach bevorzugt durch Fluor, Chlor, Methyl, Amino, Acetylamino, Dimethylamino oder Nitro substituiert sein kann.

In der europäischen Offenlegungsschrift EP-A-384 279 sind struktur-analoge Benzolsulfonamide, die atherogene Lipide senken, beschrieben.

In EP-A-4011, EP-A-255,728 und EP-A-325,245 sind Hydroxyphenyl-Verbindungen der allgemeinen Formel I als Zwischenprodukt zur Herstellung von pharmakologisch wirksamen Phenoxyalkyl-carbonsäuren beschrieben, ohne Angabe einer eigenen pharmakologischen Wirkung.

In anderen Dokumenten, wie z. B. US-A-3,737,316, FR-A-2,309,524 und FR-A-2,193,216 sind Verbindungen der Formel I mit A = Valenz beschrieben, ohne Angabe einer pharmakologischen Wirkung.

Die Verbindungen der vorliegenden Erfindung verfügen über eine starke Antioxidantienaktivität. Die Lipophilie dieser Antioxidantiengruppe bedingt, daß die Verbindungen sich im atherogenen low-density lipoprotein (LDL) anreichern und die sensitiven Bestandteile des LDL wirksam gegen reaktive Sauerstoffspezies schützen. Damit wird aber der LDL-Influx in die makrophagozytären Schaumzellen deutlich abgebremst; denn die pathologisch gesteigerte Aufnahme von atherogenem LDL in den Atheromzellen setzt dessen oxidative Modifikation voraus.

Antioxidantien sind Substanzen, die - allgemein gesehen - eine erhebliche Verzögerung der oxidativen Vorgänge bei einem zu schützenden Produkt bewirken. Ein potent antiatherosklerotisch wirksames Antioxidans ist Probucol ®, das eine hypolipidämische Wirkung bei verschiednen Tierspezies und am Menschen hat. Es ist ein sterisch gehindertes Alkylphenol, das im LDL akkumuliert. Im Tierversuch wurde gezeigt, daß Probucol die oxidative Modifikation von LDL in der Arterienwand blockiert und die Atherombildung direkt aufgrung der Antioxidansaktivität verhindert (D. Steinberg et al., Amer. J. Cardiol. 57, 16 M (1986).

Nachteile von Probucol ® liegen in der geringen Resorption der Substanz, sowie in der extrem langen Verweildauer im Körpergewebe; die Ausscheidung von Probucol erfolgt hauptsächlich über die Faeces (vgl. M.N. Cayen, Pharmacol. Ther. 29, 157 (1985)).

Ferner senken Verbindungen der Formel I die Plasmalipide indem sie die intestinale Cholesterinresorption blockieren, in Konsequenz den intrahepatischen Pool des freien Cholesterins reduzieren und die Sektretion der nahrungsabhängigen Lipoproteine aus der Leber in das Plasma entsprechend vermindern. Die Inhibition der Cholesterinresporption beruht auf einer Hemmung der Acyl-Coenzym A: -cholesterintransferase (ACAT) Reaktion. In den Enterozyten katatysiert ACAT die Veresterung des Cholesterins, die notwendig ist, um Cholesterin in Chylomikronen einzpacken und über Darmlymphe und den Ductus thoracicus in das zirkulierdende Blut einzuführen.

Die Substanzen verfügen über eine gute Resorption und inhibieren die ACAT abhängige Veresterung des freien Cholesterins nicht nur in den Enterozyten, sondern auch in den Zellen des Atheroms selbst. Sie verhindern dadurch deren schaumzellige Degeneration infolge Überladung mit Cholesterinester.

Die Verbindungen der Formel I finden aufgrund ihrer stabilisierenden Wirkung auf Lipoproteine Verwendung als Arzneimittel, insbesondere als Antiatherosclerotica.

Desweiteren wirken sie antibiotisch - besonders antibakteriell-, antiinflammatorisch, cytoprotektiv sowie antiasthmatisch. Sie können aber auch als Inhibitoren der reperfusionsabhängigen Lipidperoxidation und als Stabilisatoren des "lung surfactant factor" eingesetzt werden.

Die Herstellung der Verbindungen der Formel I ist dadurch charakterisiert, daß man ein Amin der Formel II, in welchem $R_1$, $R_2$, A und X die oben angegebene Bedeutung haben, mit Sulfonsäurechloriden der Formel III, in welchem Y ebenfalls oben angegebene Bedeutung hat, zur Reaktion bringt. Dies wird meist bei Raumtemperatur in einem chemisch inerten Lösungsmittel wie $CH_2Cl_2$, Toluol o.ä. vorzugsweise in Gegenwart eines säurebindenden Reagens, wie z.B. Pyridin, Triethylamin durchgeführt (z.B. analog F. Muth in Houben-Weyl, Bd. 9 S. 613).

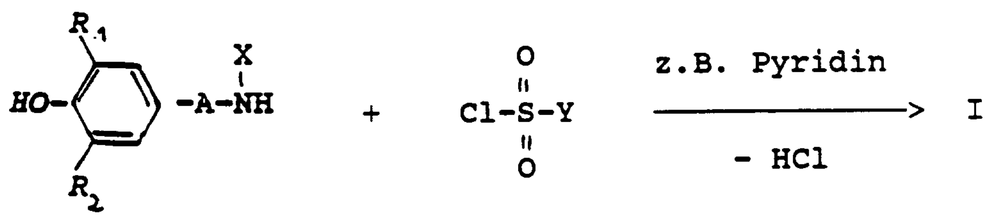


II III

Alternativ können zur Herstellung von Verbindungen der Formel I auch an der Sulfongruppe substituierte Sulfamide mit geeignet subst. Aralkylhalogeniden alkyliert werden.

Falls die so gewonnenen Verbindungen der Formel I nicht schon das Endprodukt darstellen, kann am Sulfonamid-Stickstoff ggf. mit Alkylhalogeniden (z.B. Methyliodid) zusätzlich der Rest X (z.B. $CH_3$) eingeführt werden.

Die notwendigen Ausgangsmaterialien II und III sind meist literaturbekannt, bzw. nach üblichen Verfahren analog herstellbar (detaillierte Angaben vgl. Beispiele).

Falls einzelne Reaktionsprodukte nicht in genügender Reinheit anfallen, kann die Reinigung der Rohprodukte durch Kristallisation oder Säulenchromatographie erfolgen.

Gegenstand der Erfindung sind auch neue Sulfonamide der Formel I'

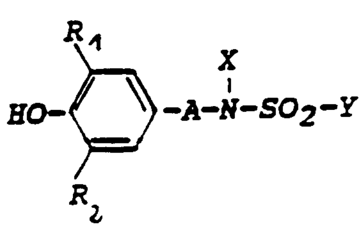

(I')

in der

$R_1$ und $R_2$ jeweils eine tert. Butylgruppe,

- A eine geradkettige Alkylenkette mit 1 bis 5 C-Atomen oder die Gruppe $-CH_2-CH(CH_3)-$
- X ein Wasserstoffatom, oder einen $C_1$-$C_4$-Alkylrest, und
- Y einen Aralkyl- oder Arylrest, wobei der Arylrest ein- bis dreifach in allen möglichen Positionen am

Ring durch Halogen, Trifluormethyl, $C_1$-$C_4$-Alkyl, Amino, $C_1$-$C_4$-Acylamino, Di($C_1$-$C_4$)-Alkylamino oder Nitro substituiert sein kann,
bedeuten,
sowie deren pharmakologisch unbedenkliche Salze.

Insbesondere sind die folgenden Verbindungen der Formel I' bevorzugt:
4-Chlorbenzolsulfo-(3,5-di-tert.butyl-4-hydroxy)-phenethylamid (Beispiel 1d)
4-Chlorbenzolsulfo-(3,5-di-tert.butyl-4-hydroxy)benzylamid (Beispiel 2)
N-Methyl-4-chlorbenzolsulfo-(3,5-di-tert.butyl-4-hydroxy)benzylamid(Beispiel 3)
Benzylsulfo-(3,5-di-tert.butyl-4-hydroxy)-benzylamid (Beispiel 5.6)
2,4,6-tris-Isopropyl-benzolsulfo-(3,5-di-tert.butyl-4-hydroxy)benzylamid (Beispiel 5.10)
2-[Benzylsulfo-(3,5-di-tert.butyl-4-hydroxy)]-phenethylamid (Beispiel 5.13)
2-[2,4,6-tris-Isopropyl-benzolsulfo-[3,5-di-tert.butyl-4-hydroxy]-phenethylamid (Beispiel 5.17)
2-[3-Trifluormethyl-benzolsulfo-(3,5-di-tert.butyl-4-hydroxy)]phenethylamid (Beispiel 5.18)
3-[2,4,6-tris-Isopropyl-benzolsulfo-[3,5-di-tert.butyl-4-hydroxy)]-phenylpropylamid (Beispiel 5.25)
4-Fluorbenzolsulfo-(3,5-di-tert.butyl-4-hydroxy)-benzylamid (Beispiel 5.27)
2-[4-Fluorbenzolsulfo-(3,5-di-tert.butyl-4-hydroxy)]-phenethylamid (Beispiel 5.28)
2-[Benzylsulfo-(3,5-di-tert.butyl-4-hydroxy)]-phenylpropylamid (Beispiel 5.32)
2-[(4-Chlorbenzol)-sulfo-(3,5-di-tert.buty-4-hydroxy)]phenylpropylamid (Beispiel 5.37)
Zur Herstellung von Salzen mit physiologisch verträglichen organischen oder anorganischen Basen wie beispielsweise Natriumhydoxid, Kaliumhydroxid, Calciumhydroxid, Ammoniumhydroxid, Methylglucamin, Morpholin, Triethylamin oder Ethanolamin können die Verbindungen der Formel I mit den entsprechenden Basen umgesetzt werden. Auch Mischungen der sauren Verbindungen mit einem geeigneten Alkalicarbonat bzw. - hydrogencarbonat kommen in Betracht.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z. B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z. B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z. B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenefalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 10 mg/kg Körpergewicht.

Bevorzugt im Sinne der Erfindung sind außer den in den Beispielen genannten Verbindungen der Formel I die folgenden:
N-Methyl-4-chlorbenzolsulfo-(3,5-di-tert.butyl-4-hydroxy)phenethylamid
N-Methyl-4-fluorbenzolsulfo-(3,5-di-tert.butyl-4-hydroxy)phenethylamid
N-Methyl-4-chlorbenzolsulfo-3-(3,5-di-tert.butyl-4-hydroxy)phenylpropylamid

Beispiel 1

a) 4-Hydroxy-3,5-di-tert.butyl-benzylchlorid
Man löst 52.8 g (0.256 mol) 2,6-Di-tert.-butylphenol in 200 ml n-Heptan, gibt 250 ml 37proz. Formalinlösung und 500 ml konz. Salzsäure zu, spült mit Stickstoff und rührt 8 h bei Rückflußtemperatur. Nach dem Abkühlen trennt man die org. Phase ab, extrahiert die wässrige Phase mit n-Heptan und wäscht die vereinigten Heptanphasen mit Wasser. Nach Trocknen mittels $Na_2SO_4$ wird i. Vak. eingedampft und der Rückstand als Rohprodukt weiterverarbeitet.
Ausbeute: praktisch quantitativ (Rohprodukt)
Lit.: Neureither, J.Org.Chem. 28, 3486 (1963)

b) 4-Hydroxy-3,5-di-tert.butylbenzylcyanid

Zu einer 80-85 °C heißen Mischung aus 38.4 g (0.71 mol) Natriumcyanid, 50 ml Wasser und 72 ml Ethanol tropft man innerhalb 1 h eine Lösung aus 104.5 g (0.41 mol) 4-Hydroxy-3,5-di-tert.butylbenzylchlorid und 155 ml Ethanol. Anschließend hält man weitere 3 h auf Rückflußtemperatur, kühlt ab und saugt anorgan. Material ab. Die flüssige Phase wird eingedampft, der Eindampfrückstand mit Wasser versetzt und mit Ether extrahiert. Man trocknet die Etherphase ($Na_2SO_4$), dampft ein und bringt durch Ligroin-Zugabe zur Kristallisation.

Ausbeute: 61.1 g (61 % d. Theorie)

Schmp.: 109-110 °C

analog Fuson und Rabjohn, Org. Synth. Vol. 25, 66.

c) 3,5-Di-tert.butyl-4-hydroxy-phenethylamin

In einem Schüttelautoklaven werden 30 g (0.12 mol) 3,5-Di-tert.butyl-4-hydroxy-benzylcyanid in 400 ml Methanol unter Tiefkühlung mit 10 g Raney-Nickel und 100 ml flüssigem Ammoniak versetzt und anschließend in einer Wasserstoffatmosphäre bei 80 °c und 140 bar 16 h hydriert. Nach Entspannen des Autoklaven wird der Katalysator abgefiltert und das Rohprodukt i. Vak. vom Lösungsmittel befreit. Den Rückstand verteilt man erneut in Ether/Wasser, trennt die Etherphase ab und trocknet über $MgSO_4$. Nach erneutem Eindampfen verbleiben grünliche, wachsartige Kristalle. Ausbeute: quantitativ (Rohprodukt).

d)

**4-Chlorbenzolsulfo-(3,5-di-tert.butyl-4-hydroxy)-phenethylamid Ia**

Zu einer Lösung von 2.49 g (10 mmol) des unter c) erhaltenen Amins in 30 ml Dichlormethan und 0.81 ml (10 mmol) Pyridin tropft man bei RT eine Lösung von 2.1 g (10 mmol) 4-Chlorbenzolsulfochlorid, gelöst in 10 ml Dichlormethan. Nach 12 h Rühren bei RT wird auf 1 H HCl gegossen, die organische Phase abgetrennt, gewaschen und mit $Na_2SO_4$ getrocknet. Nach Abziehen des Lösungsmittels i. Vak. wird der Rückstand durch Filtration über Kieselgel mit Hexan/Essigester (3:1) als Laufmittel gereinigt. Nach Einengen wird das so erhaltene Produkt aus Cyclohexan umkristallisiert.

1.4 g farblose Kristalle, Fp: 122-23 °C.

## Beispiel 2

**4-Chlorbenzolsulfo-(3,5-di-tert.butyl-4-hydroxy)benzylamid Ib**

Zu einem Gemisch aus 1.9 g (8 mmol) 3,5-Di-tert.butyl-4-hydroxy-benzylamin (hergestellt analog: Houben-Weyl, Methoden der Organischen Chemie Bd. 11/1, S. 502, aus 3,5-Di-tert.butyl-4-hydroxy-benzaldoxim) und 0.63 ml (8 mmol) Pyridin in 20 ml Dichlormethan tropft man bei RT 1.55 g (8 mmol) 4-Chlorbenzolsulfochlorid, gelöst in 10 ml Dichlormethan. Nach 24 h Rühren bei RT gießt man auf 1 N HCl, trennt die organische Phase ab, trocknet mit $Na_2SO_4$ und entfernt das Lösungsmittel i. Vak.. Das so erhaltene Rohprodukt wird aus Toluol umkristallisiert. 1.3 g farbloses Kristalle, Fp: 119-21 °C.

Beispiel 3

**N-Methyl-4-chlorbenzolsulfo-(3,5-di-tert.butyl-4-hydroxy)-benzylamid** Ic ($X = CH_3$)

Zu einer Suspension von 0.1 g (4 mmol) Natriumhydrid in 10 ml THF wurden 1.5 g (4 mmol) 4-Chlorbenzolsulfo-(3,5-di-tert.butyl-4-hydroxy)benzylamid

Ib

gegeben. Nach 30 min Rühren tropft man 0.31 ml (5 mmol) Methyliodid zu und rührt 3 h bei RT nach. Anschließend gießt man auf 1 N HCl, ethert die organische Substanz aus. Nach Trocknen und Eindampfen des Etherauszugs erhält man ein öliges Rohprodukt, das an einer Mitteldrucksäule (Kieselgel; Laufmittel Heptan/Essigester 9:1) gereinigt wird. Nach Entfernen des Lösungsmittels erstarrt das Produkt kristallin. 0.35 g gelbliche Kristalle, Fp: 140-43 ° C.

Beispiel 4

a) 3-(3,5-Di-tert.butyl-4-hydroxy)phenylpropylamin
In einem Schüttelautoklaven werden 77.8 g (0.3 mol) 3-(3,5-Di-tert.butyl-4-hydroxy)-phenylpropansäurenitril (hergestellt nach: DOS 2 240 609 (1972), Farbw. HOECHST) in 500 ml Methanol unter Tiefkühlung mit 10 g Raney-Nickel und 100 ml flüssigem Ammoniak versetzt und anschließend in einer Wasserstoffatmosphäre bei 80 ° C und 140 bar 16 h hydriert. Nach Entspannen des Autoklaven wird der Katalysator abgefiltert und das Rohprodukt i. Vak. vom Lösungsmittel befreit. Den Rückstand verteilt man erneut in Ether/Wasser, trennt die Etherphase ab und trocknet über $MgSO_4$. Nach erneutem Eindampfen verbleibt eine halbfeste Masse, die langsam völlig erstarrt.
70.2 g, grünliche Kristalle (Rohprodukt).
b)

**4-Chlorbenzolsulfo-3-(3,5-di-tert.butyl-4-hydroxy)phenyl-propylamid** Id

Eine Suspension von 3.95 g (15 mmol) des oben unter a) erhaltenen Amins in 50 ml Toluol wird zunächst mit 2.04 ml (15 mmol) Triethylamin und darauf bei RT mit 3.2 g (15 mmol) 4-Chlorbenzolsulfochlorid, gelöst in 10 ml Toluol, versetzt. Nach 12 h Rühren bei RT wird mit 50 ml Eiswasser versetzt, die organische Phase abgetrennt, getrocknet und das Lösungsmittel i. Vak. entfernt. Das Rohprodukt wird durch Filtration über Kieselgel (Hexan/Essigester 1:1 als Laufmittel) gereinigt.
1.9 g bräunliche Kristalle, Fp: 152 ° C.

Beispiel 5

In analoger Weise zu den in den Beispielen 1, 2, 3, oder 4 genannten Verbindungen erhält man (siehe Tabelle):

Tabelle 1

| Beispiel | Y | A | F.p. [°C] |
|---|---|---|---|
| 5.1 | $H_3C-(CH_2)_3-$ | Valenz | 115-18 |
| 5.2 | $(CH_3)_2CH-$ | Valenz | 160-61 |
| 5.3 | 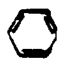 $-CH_2-$ | Valenz | 123-24 |
| 5.4 | $H_3C-(CH_2)_3-$ | $-CH_2-$ | 74-75 |
| 5.5 | $(CH_3)_2CH-$ | $-CH_2-$ | 114-15 |

Fortsetzung Tabelle 1

| Beispiel | Y | A | F.p. [°C] |
|---|---|---|---|
| 5.6 | 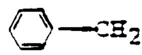$CH_2$- | $-CH_2-$ | 118-19 |
| 5.7 | $O_2N$- 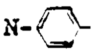 | $-CH_2-$ | 144-45 |
| 5.8 | $H_2N$- 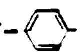 | $-CH_2-$ | Öl |
| 5.9 | $H_3COC$-N(H)- 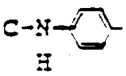 | $-CH_2-$ | 174-76 |
| 5.10 | 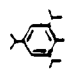 | $-CH_2-$ | 145-46 |
| 5.11 | $H_3C-(CH_2)_3-$ | $-CH_2-CH_2-$ | 109-10 |
| 5.12 | $(CH_3)_2CH-$ | $-CH_2-CH_2-$ | 81-82 |
| 5.13 | 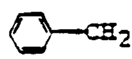$CH_2$ | $-CH_2-CH_2-$ | 163-64 |
| 5.14 | $O_2N$- 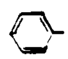 | $CH_2-CH_2-$ | 147-48 |
| 5.15 | $H_2N$- 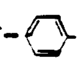 | $-CH_2-CH_2-$ | Öl |
| 5.16 | $H_3COC$-N(H)- 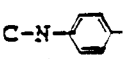 | $-CH_2-CH_2$ | 172-78 |
| 5.17 | 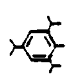 | $-CH_2-CH_2-$ | 159-60 |
| 5.18 | 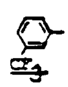 | $-CH_2-CH_2-$ | 94-95 |
| 5.19 | $H_3C-(CH_2)_3-$ | $-(CH_2)_3-$ | Öl |
| 5.20 | $(CH_3)_2CH-$ | $-(CH_2)_3-$ | Öl |
| 5.21 | 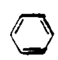 $-CH_2-$ | $-(CH_2)_3-$ | Öl |
| 5.22 | $O_2N$- 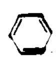 | $-(CH_2)_3-$ | Öl |

Fortsetzung Tabelle 1

| Beispiel | Y | A | F.p. [°C] |
|---|---|---|---|
| 5.23 | 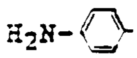 | $-(CH_2)_3-$ | 122 |
| 5.24 | 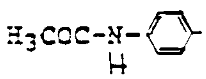 | $-(CH_2)_3-$ | 164 |
| 5.25 | 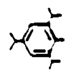 | $-(CH_2)_3$ | 144 |
| 5.26 | 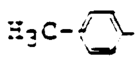 | $-(CH_2)_3-$ | 129-30 |
| 5.27 | 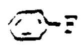 | $-CH_2-$ | 131-32 |
| 5.28 | 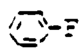 | $-CH_2-CH_2-$ | 118-20 |
| 5.29 | 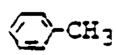 | " | 121-22 |
| 5.30 | n-Butyl- | $-CH_2-CH(CH_3)-$ | 126 |
| 5.31 | i-Propyl- | " | 113-21 |
| 5.32 | Benzyl- | " | 147-48 |
| 5.33 | 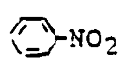 | " | 139-40 |
| 5.34 | 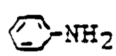 | " | 149-51 |
| 5.35 | 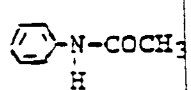 | " | 92-93 |
| 5.36 | 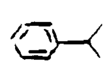 | " | 128-29 |
| 5.37 | 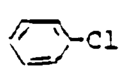 | " | 139-40 |
| 5.38 | n-Butyl | $-CH_2-(CH_3)_2C-$ | 90-91 |

Fortsetzung Tabelle 1

| Beispiel | Y | A | F.p. [°C] |
|---|---|---|---|
| 5.39 | Benzyl- | " | 143-44 |
| 5.40 | 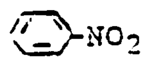 | " | 180-81 |
| 5.41 | 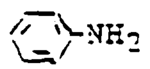 | " | 147-48 |
| 5.42 | 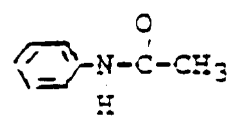 | " | 195-98 |
| 5.43 | 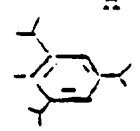 | " | 177-79 |
| 5.44 | 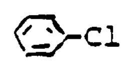 | " | 168-70 |

Beispiel 6 Phamakologische Untersuchungen

Die Verbindungen der Formel I bzw. I' sind Antioxidantien-Sulfonamidanaloga, die Hemmstoffe der Cholesterinesterbildung in der Arterienwand zur Behandlung der Arteriosklerose darstellen.

a) Die Zytogenese der Atheromatose

Hypercholesterinämie und Syndrome von Hypercholesterinämie/Hypertriglyceridämie Syndrome sind Risikofaktoren für die vorzeitige Arteriosklerose. In der Pathogenese der arteriosklerotischen Läsion selbst spielt die oxidative Modifikation von Lipoproteinen in der Arterienwand eine wichtige Rolle. Sie führt zur Freisetzung von chemotaktisch wirksamen Mediatoren und zur Wanderung von makrophagozytären Zellen aus dem Blut in die Arterienwand. In der Gefäßwand nehmen Makrophagen besonders intensiv oxidativ verändertes low-density lipoprotein (LDL) unreguliert auf. Die damit verbundene Erhöhung des Cholesterininfluxes und die Belastung des zellulären Stoffwechsels führt zu einem Anstieg des intrazellulären Cholesterins, einer Steigerung der Reacylierung des Cholesterins und Speicherung der Cholesterinester, die wiederum zur Ausbildung großer cytoplasmatischer Cholesterinesterdepots und zur Degeneration dieser Makrophagen ("Schaumzellen") führen. Die Schaumzellen sind das zelluläre Substrat des Atheroms und verursachen die makroskopisch sichtbaren Fettstreifen, aus denen sich dann die fibrösen Plaqueläsionen bei Fortschreiben der Erkrankung entwickeln.

b) Die Grundlagen für eine antiatherosklerotische Therapie bei Fettstoffwechselstörungen

Eine an die Kausalzusammenhänge orientierte antiatherosklerotische Therapie fußt idealerweise auf mindestens drei Säulen: 1. einer Senkung pathologisch erhöhter Lipidspiegel im Plasma durch Diät und/oder Pharmaka, 2. einem wirksamen Antioxidantienschutz besonders des stark atherogenen LDL zur Verminderung des oxidativen LDL-Katabolismus im Atherom und 3. einer Hemmung der Cholesterinesterbildung in den Schaumzellen der fettstreifigen Frühläsionen.

Einige Senker des Serum-Cholesterins wirken, indem sie den hepatischen Pool des freien Cholesterins kontrahieren und den Katabolismus des atherogenen LDL-Cholesterins erhöhen: Ionenaustauscher interferieren z. B. mit dem enterhepatischen Cholesterinkreislauf, führen zur Depletion der Gallensäuren in den Hepatozyten und stimulieren die LDL-Cholesterinaufnahme aus dem Plasmakompartment durch eine vermehrte hepatische Aufnahme von Cholesterin über LDL und die hepatischen Apo B,E-Rezeptoren (LDL-Rezeptoren). Ähnlich wirken die Cholesterinsynthese-blocker, z. B. Lovastatin. Sie vermindern das hepatische Cholesterin und bedingen ebenfalls eine gesteigerte Aufnahme von LDL-Cholesterin aus dem Plasma über eine Vermehrung der LDL-Rezeptoren.

Auch Hemmer der Cholesterinresorption bedingen eine Absenkung des hepatischen Cholesterinpools, indem sie die exogene diätetische Belastung durch Cholesterin reduzieren. Beispiele hierfür sind die sogenannten Acyl CoA: cholesterinacyltransferase (ACAT)-Hemmer, die in den Enterozyten den Cholesterinefflux via Chylomikronen durch die Hemmung der Veresterung freien Cholesterins unterbinden. In der Leber wird durch diese Hemmer der Cholesterinesterbildung zusätzlich das freie Cholesterin erhöht und die endogene hepatische Cholesterinbiosynthese durch Endprodukt-Hemmung indirekt gehemmt. In den Schaumzellen des Atheroms wird durch die Blockade der Cholesterinesterbildung erhöht freies Cholesterin in den "Reverse Cholesterol Transport pathway" der high-density lipoproteins (HDL) eingebracht, daraus entsteht ein zusätzlicher direkter antiatherogener Effekt.

Antioxidantien sind Substanzen, die - allgemein gesehen - eine erhebliche Verzögerung der oxidativen Vorgänge bei einem zu schützenden Produkt bewirken. Ein potent antiatherosklerotisch wirksames Antioxidans ist Probucol, das neben der Antioxidanswirkung eine hypolipidämische Wirkung bei verschiedenen Tierspezies und am Menschen hat. Es ist ein sterisch gehindertes Alkylphenol, das im LDL akkumuliert. Im Tierversuch wurde gezeigt, daß Probucol die in den atherosklerotischen Läsionen der Arterienwand sehr stark erhöhte oxidative Modifikation von LDL blockiert und die Atherombildung massiv hemmt aufgrund des durch die Antioxidantien abgebremsten Katabolismus von LDL-Cholesterin in den Schaumzellen.

c) Aminosubstituierte Phenole mit antiatherogener Wirkung

Die Verbindungen der Formel I und I' erfüllen alle drei genannten Voraussetzungen für ein wirksames Arzneimittel gegen die vorzeitige Ateromatose und die progressive Arteriosklerose, da sie hypolipidämisch wirken (Tabelle 1) und durch Hemmung der Cholesterinesterbildung (Tabelle 2) wie aufgrund der Antioxidantienaktivität im LDL (Tabelle 2) und gegenüber von zellulären Lipoxygenasen (Tabelle 2) eine direkte antiatherosklerotische Aktivität im Atherom entfalten.

d) Dosisabhängiger Effekt der Verbindung aus Beispiel 5.28 auf den Serum-Cholesterinspiegel in Ratten mit diätetisch induzierter Hypercholesterinämie.

Methode:

220-250 g schwere SD Ratten erhielten eine Standarddiät, die mit 2 % Cholesterin/0,2 % Cholsäure angereichert wurde, über 4 Tage. Die Tiere wurden täglich zwischen 8:00 und 9:00 sondiert und erhielten die angegebene Einmal-Dosis über die Schlundsonde in 2 ml Pflanzenöl. Bestimmung des S-Cholesterins am Tag 5 (Testkit der Fa. Boehringer Mannheim).

Tabelle 1

| Dosierung (mg/kg/d) | S-Cholesterin (mg %) | % Kontrollen | Statistik |
|---|---|---|---|
| 0 | 248 +/- 36 | 100 | - |
| 5 | 187 +/- 23 | 75 | p < 0,05 |
| 15 | 120 +/- 15 | 48 | p < 0,05 |
| 50 | 114 +/- 14 | 46 | p < 0,02 |
| 100 | 84 +/- 8 | 33 | p < 0,01 |
| Mittelwerte +/- SEM, n=6 Tiere/Gruppe, Statistik: U-Test | | | |

e) In-vitro-Wirkung der Verbindungen der Formel I und I'

Hemmung der Cholesterinesterbildung in Makrophagen (nach Huber et al. (1990) Free Rad. Res. Comms. 8, 167-173):

Humanplasma-LDL wurde durch sequenzielle Ultrazentrifugation bei präselektierten Dichten aus dem Plasma gesunder männlicher Nichtraucher isoliert und für 5 h bei 37° C in F-10 Medium + 1 µM Cu(II) zur Depletion seiner lipophilen Antioxidantien inkubiert. Die Bildung von Cholesterinestern in P 388 D.1 Makrophagen unter Einfluß von konditioniertem human Plasma-LDL wurde nach 18 h Inkubation der Zellen (2 x $10^6$/ml) in F-10 Medium + 1 µ M Cu(II) + 50 µg/ml LDL + 5 % FCS nach Extraktion der Neutralfette dünnschichtchromatographisch gemessen.

Referenzsübstanz: Probucol, 10 µM Hemmung der Cholesterbildung 25-42 % (n=7).

Stabilisierung von Plasma-LDL (nach Huber et al. (1990) Free Rad. Res. Comms. 8, 167-173):

LDL (125 µg Protein/ml) wurde in 20 mM Tris-Cl pH 8, 150 mM NaCl, 10 µM $CuCl_2$ mit und ohne

Testverbindung (gelöst in Äthanol, Äthanolendkonzentration 0,5 %) bei 42° C oxidiert. Es wurden jeweils 3 Moleküle Testsubstanz pro LDL-Partikel eingesetzt. Die Oxidation wurde kontinuierlich photometrisch bei A 234 durch die Messung der Dienbildung verfolgt. Die sogenannte "lag Phase" ist als dasjenige Zeitintervall definiert, das sich aus dem Schnittpunkt der Tangente der Kurve während der Propagationsreaktion der Lipidperoxidation und er Zeitachse ergibt.

Referenzsubstanz: Probucol, 3 Moleküle/LDL-Partikel verlängern die lag-Phase von LDL um 10 %.

Hemmung der Sojabohnenlipoxygenase (15-Lipoxygenase) am LDL-Substrat (nach Cathcart et al. (1991) J. Lipid Res. 32, 63-70):

LDL (100 µg Protein/ml) wurde in 20 mM Tris-Cl pH 8,3, 150 mM NaCl mit und ohne Testsubstanz (gelöst in Äthanol, Endkonzentration des Äthanol 0,5 %) mit 5000 U/2ml Sojabohnen-Lipoxygenase (Sigma, München) bei 37° C inkubiert: Die Oxidation wurde durch Messung der Diene kontinuierlich bei A 234 verfolgt. Als $IC_{50}$-Wert wird diejenige Konzentration der Testverbindung angegeben, die zur Halbierung der im Testansatz über die Zeit linearen Dienbildung führt.

Referenzsubstanz: Probucol, $IC_{50}$-Wert = 20 µM.

Tabelle 2

| Verbindung Bsp.) | Hemmung der Cholesterinesterbildung in Macrophagen (P388D.1) | | Stabilisierung von Plasma LDL: Verlängerung d. "lag-Phase" % | Hemmung der 15-Lipoxygenase vermitt. LDL-Oxidation $IC_{50}$ (µM) |
|---|---|---|---|---|
| | $IC_{50}$ (µM) | 10 µM % | | |
| 2 | 2,50 | -78,7 | 44 | 0,2 |
| 3 | | -54,9 | 40 | 2 |
| 4b | | -49,2 | | 0,2 |
| 5.10 | 7 | -60,3 | 99 | 5 |
| 5.17 | | -78,0 | 50 | 3 |
| 5.19 | | -43,8 | | 0,09 |
| 5.28 | 1,20 | -92,6 | 25 | <0,1 |
| 5.31 | | -33.9 | 18 | 0,2 |

## Patentansprüche

**1.** Arzneimittel, enthaltend mindestens ein Sulfonamid der allgemeinen Formel I

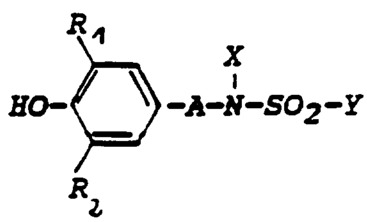

I

in der

$R_1$ und $R_2$, die gleich oder verschieden sein können,

ein Wasserstoffatom oder einen geradkettigen oder verzweigten Alkylrest mit 1 bis 4 C-Atomen,

A eine geradkettige oder verzweigte Alkylenkette mit 1 bis 5 C-Atomen,

X ein Wasserstoffatom, oder einen $C_1$-$C_4$-Alkylrest,

und

Y einen geradkettigen oder verzweigten Alkylrest mit 1 bis 5 C-Atomen, einen Aralkyl- oder Arylrest, wobei der Arylrest ein- bis dreifach in allen möglichen Positionen am Ring durch Halogen, Trifluormethyl, $C_1$-$C_4$-Alkyl, Amino, $C_1$-$C_4$-Acylamino, Di($C_1$-$C_4$)-Alkylamino, oder Nitro substituiert sein kann,

bedeuten,

mit der Maßgabe, daß A auch Valenz bedeuten kann, wenn kein Y keinen Arylrest darstellt, sowie deren pharmakologisch unbedenkliche Salze neben üblichen Träger- und Hilfsstoffen.

**2.** Neue Sulfonamide der Formel I'

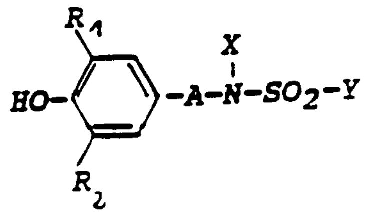

I'

in der

| | |
|---|---|
| $R_1$ und $R_2$ | jeweils eine tert. Butylgruppe, |
| A | eine geradkettige Alkylenkette mit 1 bis 5 C-Atomen oder die Gruppe $-CH_2-CH(CH_3)-$ |
| X | ein Wasserstoffatom, oder einen $C_1$-$C_4$-Alkylrest, und |
| Y | einen Aralkyl- oder Arylrest, wobei der Arylrest ein- bis dreifach in allen möglichen Positionen am Ring durch Halogen, Trifluormethyl, $C_1$-$C_4$-Alkyl, Amino, $C_1$-$C_4$-Acylamino, Di($C_1$-$C_4$)-Alkylamino oder Nitro substituiert sein kann, bedeuten, sowie deren pharmakologisch unbedenkliche Salze. |

**3.** Verbindungen gemäß Anspruch 2, ausgewählt aus der Gruppe
4-Chlorbenzolsulfo-(3,5-di-tert.butyl-4-hydroxy)-phenethylamid
4-Chlorbenzolsulfo-(3,5-di-tert.butyl-4-hydroxy)benzylamid
N-Methyl-4-chlorbenzolsulfo-(3,5-di-tert.butyl-4-hydroxy)benzylamid
Benzylsulfo-(3,5-di-tert.butyl-4-hydroxy)-benzylamid
2,4,6-tris-Isopropyl-benzolsulfo-(3,5-di-tert.butyl-4-hydroxy)-benzylamid
2-[Benzylsulfo-(3,5-di-tert.butyl-4-hydroxy)]-phenethylamid
2-[2,4,6-tris-Isopropyl-benzolsulfo-[3,5-di-tert.butyl-4-hydroxy]-phenethylamid
2-[3-Trifluormethyl-benzolsulfo-(3,5-di-tert.butyl-4-hydroxy)]phenethylamid
3-[2,4,6-tris-Isopropyl-benzolsulfo-[3,5-di-tert.butyl-4-hydroxy)]-phenylpropylamid
4-Fluorbenzolsulfo-(3,5-di-tert.butyl-4-hydroxy)-benzylamid
2-[4-Fluorbenzolsulfo-(3,5-di-tert.butyl-4-hydroxy)]phenethylamid
2-[Benzylsulfo-(3,5-di-tert.butyl-4-hydroxy)]-phenylpropylamid
2-[(4-Chlorbenzol)-sulfo-(3,5-di-tert.buty-4-hydroxy)]phenylpropylamid

**4.** Verfahren zur Herstellung von Verbindungen der Formel I'

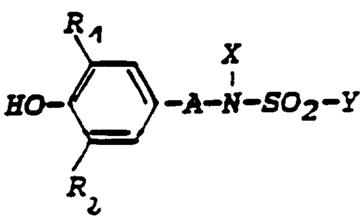

I'

in der

| | |
|---|---|
| $R_1$ und $R_2$ | jeweils eine tert. Butyl-Gruppe, |
| A | eine geradkettige Alkylenkette mit 1 bis 5 C-Atomen oder die Gruppe |

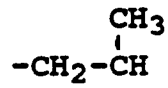

X ein Wasserstoffatom, oder einen $C_1$-$C_4$-Alkylrest,

und

Y einen Aralkyl- oder Arylrest, wobei der Arylrest ein- bis dreifach in allen möglichen Positionen am Ring durch Halogen, Trifluormethyl, $C_1$-$C_4$-Alkyl, Amino, $C_1$-$C_4$-Acylamino, Di($C_1$-$C_4$)-Alkylamino, oder Nitro substituiert sein kann,

bedeuten,

sowie deren pharmakologisch unbedenkliche Salze,

dadurch gekennzeichnet, daß man in an sich bekannter Weise entweder

a) ein Amin der Formel II

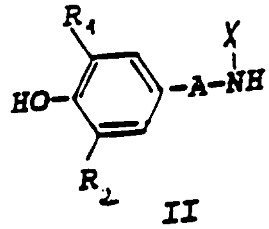

II

in der

$R_1$, $R_2$, A und X die angegebene Bedeutung haben mit einem Sulfochlorid der Formel III

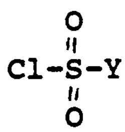

III

in der

Y die angegebene Bedeutung hat, in Gegenwart eines säurebindenden Reagenz umsetzt,

oder

b) für den Fall, daß A eine Alkylenkette darstellt, ein Sulfonamid der Formel IV

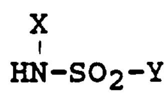

IV

in der

X und Y die angegebenen Bedeutungen haben,

mit einem Aralkylhalogenid der Formel V

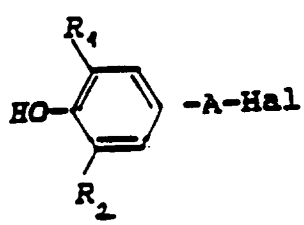

V

in der

$R_1$ und $R_2$ die angegebenen Bedeutungen haben, A eine Alkylkette und Hal ein Halogenatom darstellen umsetzt,
und anschließend, für den Fall, daß X Wasserstoff bedeutet, die erhaltenen Verbindungen in üblicher Weise in andere Verbindungen mit X = Alkyl überführt, sowie gewünschtenfalls die erhaltenen Verbindungen in pharmakologisch verträgliche Salze überführt.

**5.** Verwendung von Verbindungen der Formel I gemäß Anspruch 1, 2 oder 3 zur Herstellung von Arzneimitteln mit antiatherosklerotischer Wirkung.

## Claims

**1.** Medicaments containing at least one sulphonamide of the general formula I

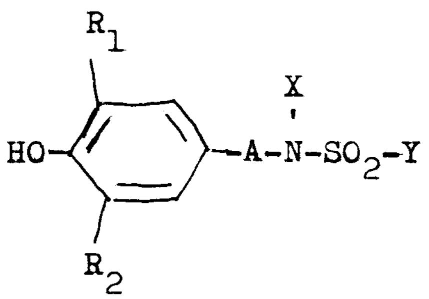

I

in which $R_1$ and $R_2$, which can be the same or different, signify a hydrogen atom or a straight-chained or branched alkyl radical with 1 to 4 C-atoms, A a straight-chained or branched alkylene chain with 1 to 5 C-atoms, X a hydrogen atom or a $C_1$-$C_4$-alkyl radical and Y a straight-chained or branched alkyl radical with 1 to 5 C-atoms, an aralkyl or aryl radical, whereby the aryl radical can be substituted one to three times in all possible positions on the ring by halogen, trifluoromethyl, $C_1$-$C_4$-alkyl, amino, $C_1$-$C_4$-acylamino, di-($C_1$-$C_4$)-alkylamino or nitro, with the proviso that A can also signify a valency when Y does not represent an aryl radical, as well as their pharmacologically acceptable salts, besides usual carrier and adjuvant materials.

**2.** New sulphonamides of the formula I'

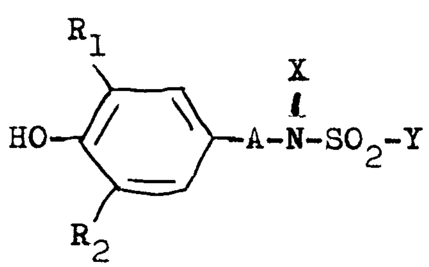

I'

in which $R_1$ and $R_2$ each signify a tert.-butyl group, A a straight-chained alkylene chain with 1 to 5 C-atoms or the group $-CH_2-CH(CH_3)-$, X a hydrogen atom or a $C_1$-$C_4$-alkyl radical and Y an aralkyl or aryl radical, whereby the aryl radical can be substituted one to three times in all possible positions on the ring by halogen, trifluoromethyl, $C_1$-$C_4$-alkyl, amino, $C_1$-$C_4$-acylamino, di-($C_1$-$C_4$)alkylamino or nitro, as well as their pharmacologically acceptable salts.

**3.** Compounds according to claim 2, selected from the group
4-chlorobenzenesulpho-(3,5-di-tert.-butyl-4-hydroxy)phenethylamide
4-chlorobenzenesulpho-(3,5-di-tert.-butyl-4-hydroxy)benzylamide
N-methyl-4-chlorobenzenesulpho-(3,5-di-tert.-butyl-4-hydroxy)-benzylamide
benzylsulpho-(3,5-di-tert.-butyl-4-hydroxy)-benzylamide
2,4,6-tris-isopropylbenzenesulpho-(3,5-di-tert.-butyl-4-hydroxy)-benzylamide
2-[benzylsulpho-(3,5-di-tert.-butyl-4-hydroxy)]phenethylamide

2-[2,4,6-tris-isopropylbenzenesulpho-[3,5-di-tert.-butyl-4-hydroxy]-phenethylamide
2-[3-trifluoromethylbenzenesulpho-(3,4-di-tert.-butyl-4-hydroxy)]-phenethylamide
3-[2,4,6-tris-isopropylbenzenesulpho-[3,5-di-tert.-butyl-4-hydroxy)]-phenylpropylamide
4-fluorobenzenesulpho-(3,5-di-tert.-butyl-4-hydroxy)benzylamide
2-[4-fluorobenzenesulpho-(3,5-di-tert.-butyl-4-hydroxy)]-phenethylamide
2-[benzylsulpho-(3,5-di-tert.-butyl-4-hydroxy)]phenylpropylamide
2-[(4-chlorobenzene)-sulpho-(3,5-di-tert.-butyl-4-hydroxy)]-phenylpropylamide.

**4.** Process for the preparation of compounds of the formula I'

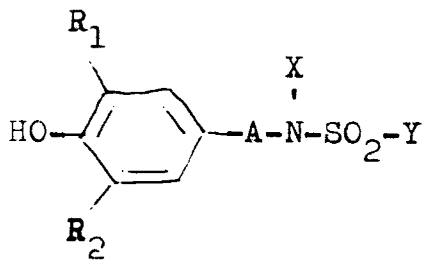

I'

in which $R_1$ and $R_2$ each signify a tert.-butyl group, A a straight-chained alkylene chain with 1 to 5 C-atoms or the group

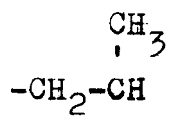

X a hydrogen atom or a $C_1$-$C_4$-alkyl radical and Y an aralkyl or aryl radical, whereby the aryl radical can be substituted one to three times in all possible positions on the ring by halogen, trifluoromethyl, $C_1$-$C_4$-alkyl, amino, $C_1$-$C_4$-acylamino, di-($C_1$-$C_4$)-alkylamino or nitro, as well as of their pharmacologically acceptable salts, characterised in that, in per se known manner, one either

a) reacts an amine of the formula II

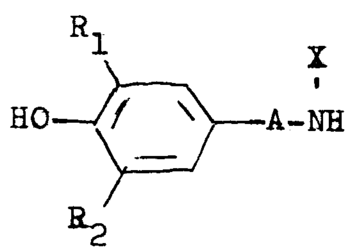

II

in which $R_1$, $R_2$, A and X have the given meaning, with a sulphochloride of the formula III

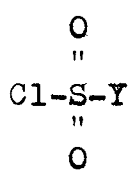

III

in which Y has the given meaning, in the presence of an acid-binding agent, or

b) for the case that A represents an alkylene chain, reacts a sulphonamide of the formula IV

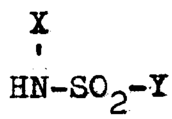

IV

in which X and Y have the given meanings, with an aralkyl halide of the formula V

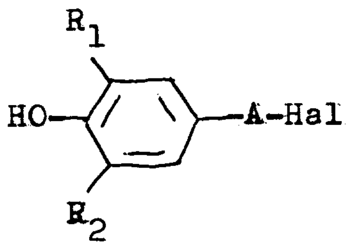

V

in which $R_1$ and $R_2$ have the given meanings, A represents an alkyl chain and Hal a halogen atom, and subsequently, for the case that X signifies hydrogen, converts the compounds obtained in the usual way into other compounds with X = alkyl, as well as, if desired, converts the compounds obtained into pharmacologically acceptable salts.

**5.** Use of compounds of the formula I according to claim 1, 2 or 3 for the production of medicaments with antiatherosclerotic action.

**Revendications**

**1.** Médicament contenant au moins un sulfamide de formule I

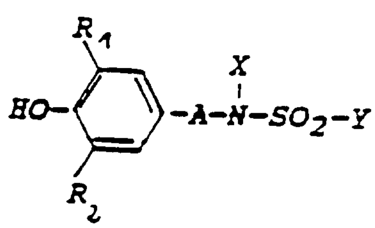

I

dans laquelle

$R_1$ et $R_2$, qui peuvent être identiques ou différents, représentent un atome d'hydrogène ou un reste alkyle à chaîne linéaire ou ramifiée, ayant 1 à 4 atomes de carbone,

A représente un reste alcényle à chaîne linéaire ou ramifiée, ayant 1 à 5 atomes de carbone,

X représente un atome d'hydrogène ou un reste alkyle en $C_1$-$C_4$,

et

Y représente un reste alkyle à chaîne linéaire ou ramifiée, ayant 1 à 5 atomes de carbone, un reste aralkyle ou aryle, le reste aryle pouvant être substitué une à trois fois, dans toutes les positions possibles du cycle, par un atome d'halogène, un groupe trifluorométhyle, alkyle en $C_1$-$C_4$, amino, acylamino en $C_1$-$C_4$, di-(alkylamino en $C_1$-$C_4$) ou nitro,

étant entendu que A peut également représenter une valence lorsque Y ne représente par un groupe aryle,

ainsi que leurs sels pharmacologiquement acceptables, en plus des supports et adjuvants usuels.

**2.** Nouveaux sulfamides de formule I'

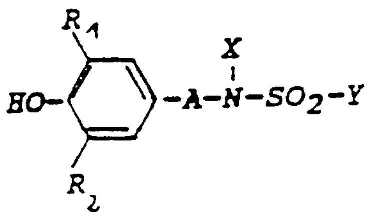

I'

dans laquelle

$R_1$ et $R_2$ représentent chacun un groupe tert.-butyle,

A représente un reste alcényle à chaîne linéaire ou ramifiée, ayant 1 à 5 atomes de carbone, ou le groupe $-CH_2-CH(CH_3)-$

X représente un atome d'hydrogène ou un reste alkyle en $C_1$-$C_4$, et

Y représente un reste aralkyle ou aryle, le reste aryle pouvant être substitué une à trois fois, dans toutes les positions possibles du cycle, par un atome d'halogène, un groupe trifluorométhyle, alkyle en $C_1$-$C_4$, amino, acylamino en $C_1$-$C_4$, di-(alkylamino en $C_1$-$C_4$) ou nitro,

ainsi que leurs sels pharmacologiquement acceptables.

**3.** Composés selon la revendication 2, choisis dans le groupe constitué par :
4-chlorobenzènesulfo-(3,5-di-tert.-butyl-4-hydroxy)-phénéthylamide
4-chlorobenzènesulfo-(3,5-di-tert.-butyl-4-hydroxy)-benzylamide
N-méthyl-4-chlorobenzènesulfo-(3,5-di-tert.-butyl-4-hydroxy)-benzylamide
benzènesulfo-(3,5-di-tert.-butyl-4-hydroxy)-benzylamide
2,4,6-tri-isopropyl-benzènesulfo-(3,5-di-tert.-butyl-4-hydroxy)-benzylamide
2-[benzènesulfo-(3,5-di-tert.-butyl-4-hydroxy)]-phénéthylamide
2-[2,4,6-tri-isopropyl-benzènesulfo-(3,5-di-tert.-butyl-4-hydroxy)]-phénéthylamide
2-[3-trifluorométhyl-benzènesulfo-(3,5-di-tert.-butyl-4-hydroxy)]-phénéthylamide
3-[2,4,6-tri-isopropyl-benzènesulfo-(3,5-di-tert.-butyl-4-hydroxy)]-phénylpropylamide
4-fluorobenzènesulfo-(3,5-di-tert.-butyl-4-hydroxy)-benzylamide
2-[4-fluorobenzènesulfo-(3,5-di-tert.-butyl-4-hydroxy)]-phénéthylamide
2-[benzènesulfo-(3,5-di-tert.-butyl-4-hydroxy)]-phénylpropylamide
2-[(4-chlorobenzène)-sulfo-(3,5-di-tert.-butyl-4-hydroxy)]-phénylpropylamide.

**4.** Procédé de préparation de composés de formule I'

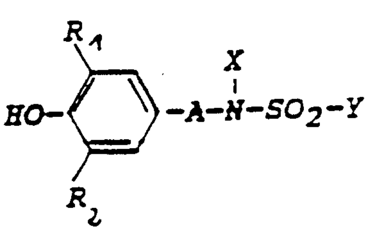

I'

dans laquelle

$R_1$ et $R_2$ représentent chacun un groupe tert.-butyle,

A représente un reste alcényle à chaîne linéaire, ayant 1 à 5 atomes de carbone, ou le groupe

$-CH_2-CH(CH_3)-$

X représente un atome d'hydrogène ou un reste alkyle en $C_1$-$C_4$, et

Y représente un reste aralkyle ou aryle, le reste aryle pouvant être substitué une à trois

fois, dans toutes les positions possibles du cycle, par un atome d'halogène, un groupe trifluorométhyle, alkyle en $C_1$-$C_4$, amino, acylamino en $C_1$-$C_4$, di-(alkylamino en $C_1$-$C_4$) ou nitro,

ainsi que leurs sels pharmacologiquement acceptables,

caractérisé en ce que de manière connue en soi, on fait réagir soit

a) une amine de formule II

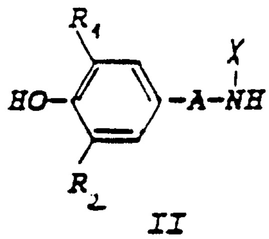

II

dans laquelle

$R_1$, $R_2$, A et X ont les significations mentonnées plus haut, avec un sulfochlorure de formule III

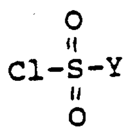

III

dans laquelle

Y à la signification indiquée ci-dessus, en présence d'un réactif fixant les acides, soit

b) dans le cas où A représente une chaîne alcényle, on fait réagir un sulfamide de formule IV

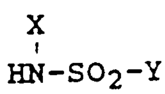

IV

dans laquelle

X et Y ont les significations indiquées ci-dessus,

avec un halogénure d'aralkyle de formule V

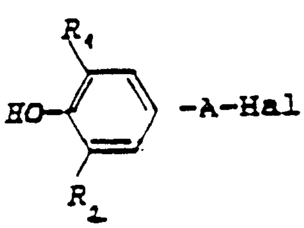

V

dans laquelle

$R_1$ et $R_2$ ont les significations mentionnées ci-dessus, A représente un groupe alkyle et Hal, un atome d'halogène,

et ensuite, dans le cas où X représente un atome d'hydrogène, on transforme les composés obtenus de manière usuelle en d'autres composés dans lesquels X = alkyle, ainsi qu'éventuellement, on transforme les composés obtenus en leurs sels pharmacologiquement acceptables.

**5.** Utilisation de composés de formule I, selon la revendications 1, 2 ou 3, pour la préparation de médicaments ayant une activité anti-athérosclérose.